(19) Europäisches Patentamt
European Patent Office
Office européen des brevets

(11) **EP 2 498 080 B1**

(12) **EUROPEAN PATENT SPECIFICATION**

(45) Date of publication and mention
of the grant of the patent:
**02.10.2013 Bulletin 2013/40**

(51) Int Cl.:
***G01N 22/04*** *(2006.01)*      ***G01N 33/38*** *(2006.01)*

(21) Application number: **11466024.4**

(22) Date of filing: **02.09.2011**

(54) **Apparatus for measuring of moisture parameters of porous materials and method for examination of moisture spreading in porous materials**

Vorrichtung zum Messen von Feuchteparametern poröser Materialien und Methode zur Prüfung der Feuchtewanderung in porösen Materialien

Dispositif pour mesurer des paramètres d'humidité dans des matériaux poreux et méthode pour examiner le transport d'humidité dans des matériaux poreux

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**12.09.2012 Bulletin 2012/37**

(73) Proprietor: **Vysoké Ucení Technické V Brne**
**602 00 Brno (CZ)**

(72) Inventors:
• **Skramlik, Jan**
**621 00 Brno (CZ)**
• **Novotny, Miloslav**
**635 00 Brno (CZ)**
• **Fuciman, Ondrej**
**625 00 Brno (CZ)**
• **Suhajda, Karel**
**664 01 Bilovice Nad Svitavou (CZ)**

(74) Representative: **Malusek, Jiri**
**Kania, Sedlak, Smola**
**Mendlovo namesti 1 a**
**603 00 Brno (CZ)**

(56) References cited:
**EP-A2- 1 918 696**

• **Jan Skramlík: "7. Návrh sestavy merící linky" In: "Vlhkost v dutinách stavebních konstrukcí (dissertation)", 1 January 2009 (2009-01-01), XP55028880, * the whole document ***
• **WITTIG G ET AL: "Untersuchungen mit Mikrowellen zur Feuchtewanderung in Baustoffproben", BAUPHYSIK, ERNST & SOHN VERLAG. BERLIN, DE, vol. 2, no. 14, 1 January 1992 (1992-01-01), pages 45-49, XP009096438, ISSN: 0171-5445**
• **WITTIG G ET AL: "Untersuchungen an Baustoffproben mit Mikrowellen. I, Versuchsmethodik, Messergebnisse an Zementm rtelproben & II, Messergebnisse an Ziegeln", MATERIALPRUEFUNG, HANSER, MUENCHEN, DE, vol. 33, no. 4 & 5, 1 January 1991 (1991-01-01), pages 106-111,143, XP009097281, ISSN: 0025-5300**
• **"Seznam publikací", , 1 January 2012 (2012-01-01), XP55030214, Retrieved from the Internet: URL:http://synchro.fce.vutbr.cz/cdb/ publik ace/list.asp?per_id=1178 [retrieved on 2012-06-18]**

**Description**

Background of invention

The invention concerns the method for monitoring of moisture spreading in porous materials, in particular based on principle of use of the electromagnetic microwave radiation and the apparatus for its realization.

State of the art

A dominant type of realised wall building constructions is a brick construction made by pieces of a building material joined by mortar. Lot of building malfunctions is caused and folowed by influence of an undesirable moisture appearence. Current trends in the civil engineering are directed to the use of new and light building materials with the strong accent on failure-free function of building constructions, in particular on a heat-insulation character of sidings. Determination of specific parameters of materials for precise definition of the moisture behavior in building constructions is unsufficiently solved item in case of study of features of the porous building materials. The crucial value is the moisture capillary conductivity coefficient, which is derived from the measuring of the saturation and desaturation curve (moistening and drying process) according to motion of a moisture profile in a nonstationary state (i.e. before reaching of full moisture state) during its moistening.

The gravimetric method is usually used, which is a fundamental method to find out the volume of the moisture in a material. An expression of a single curve for each single specimen, impreciseness of the results due to manipulation with the specimen and low frequency of detected values due to fracturing of the specimens of materials (lengths about 20 mm) are disadvantageously. Consequently to that, only the Matan's method is possible to be applied as a gravimetric method.

A nondestructive method allows to obtain significantly higher frequency of more accurate outputs (c. each 3 mm) and elimination of the losses due to the manipulation with the specimen. Moreover, more outputs of measurements are continuously scaned and electronically evaluated without the influence of a fault due to the human factor.

The application of a nondestructive method based on electromagnetic radiation is in comparison to the method NMR (which is applied in medicine), easier affordable and also safer with regard to the prevention against the dangerous radiation.

The moisture characteristics of porous materials is still not exactly explored field with respect to its determination for practical purposes in a building practice. The problem is delt by the Czech norme CSN 15026 (2007), based on the determination of presure differences, and by CSN EN ISO 15148 (2004), which mentions only an information with recommendation to use the measurement by the NMR method, without the description of a method how to determinate input values to enumerate the capillary conductivity coefficient.

The moisture curves gained by nondestructive method measured on previous experimentaly constructed measuring apparatus are applicable for determination of the capillary conductivity coefficient, as a characteristic moisture parameter for expression of the moisture characteristics of porous materials to use it for the valuations of building materials according to the thermal technical characteristics.

The method for determination of the specific moisture parameter of the capillary conductivity coefficient is based on known physical principles. By substitution of the Lykovov's formula for density of the moisture flow into the equation of continuity we get:

$$\frac{\partial u}{\partial t} = \frac{\partial}{\partial x}\left(\kappa(u)\frac{\partial u}{\partial x}\right)$$

where u is a function of moisture weight of the specimen
x is a position in the length of the specimen
K is the capillary conductivity coefficient to be found

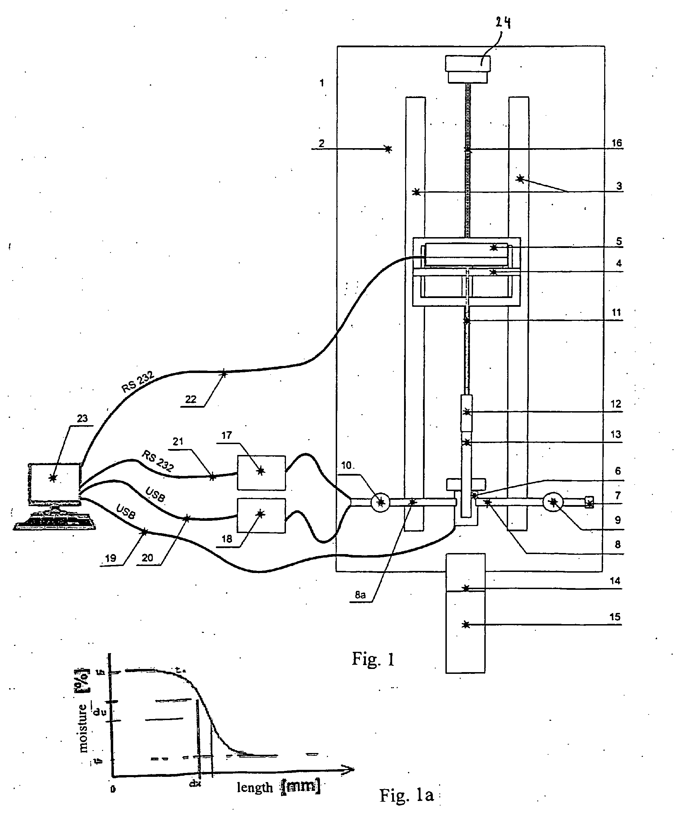

Fig. 1 a presents the moisture curve, which creates the moisture forehead of the moisture spreading profile in the material.

A half-line with a border condition,

$$u(0,t) = u_1$$

where $u_1$ is the maximum moisture (comment: the maximum saturation is not possible to achieve - the rest is a structure)

and an initiative condition,

$$u(x,0) = u_2$$

where $u_1$ is moisture obtained in time
$u_2$ is stationary moisture (as an initiative moisture provided that the material is not completely dry)

is possible for x>0 to transform by the Boltzmann transformation by its substitution into the original equation and by sequential derivation:

$$\kappa(\omega(x)) = \frac{1}{u'(x).2\sqrt{t}} \cdot \frac{1}{\sqrt{t}} \cdot \int_{x}^{\infty} \xi \, u'(\xi) d\xi \qquad [4]$$

where $\omega$ is a new variable
u is moisture weight of the specimen's material
$u_1$ is relative moisture of the specimen's material
x is a position in length of the specimen
t is time interval of the measurement

and for the new variable $\omega$ is a rule:

$$\omega(0) = u_1$$

and

$$\omega(\infty) = u_2$$

when $\sqrt{t}$ will convergate to zero.

The above mentioned method is based on that, that the measured specimen with defined length is moistured on its forehead and water is allowed to spread into the specimen during a specific time period. After that, the specimen is divided in to several segments - specimens, which have different moisture extend. Moisture is measured in each segment and then the curve of moisture spreading in the specimen is determined on base of these results. Generally, this method is strained by impreciseness during the manipulation with the specimen and this method is practicaly unusable for repetitive measurement of the specimen to obtain the capillary conductivity coefficient by the integral method.

For measuring of the moisture it was possible to use the measuring method of the neutron radiation absorption, which is based on the principle of nuclear magnetic resonance. However, such method is so expensive and requires dimensional mechanical devices and it is also difficult to provide sufficient protection against the radiation danger. So this method is unsuitable in the building practice.

In CZ 301475 B1 the method of monitoring of moisture spreading in porous materials is presented, where after hang-up the specimen on the scale the container, which is full of liquid, is placed below it in such a way, that the forehead of the speciment is moistured due to the contact with the liquid level, whereas microwave radiation is brought to the specimen by waveguides, which is allowed to go through the specimen near to its forehead. After activation of a displacement device the waveguides are continuously displaced upwards in the length of the monitored specimen and moisture in the saturated area is continuously measured from the forehead of the specimen upwards, whereas changes of the passed radiation and changes of specimen's weight are measured in time and results are quantificated and further evaluated by connected computer. The device for this method comprises a displacement device to change the height of the container, respectively the liquid level, whereas the hanger is arranged with the upper part on the digital scale, whereas above the container the transmitting waveguide is arranged and on the edge, which is reversed to the container, source of microwave radiation is arranged, whereas from the other side, opposite to the transmitting waveguide, receiving waveguide is arranged, whereas both waveguides, as one unit, are displaceably arranged on a bearing frame by positioning mechanism. The waveguides are made of hollow metal profile and the specimen is arranged between mutualy turned ends of the waveguides, whereas the receiving waveguide is on the side reversed to the container provided by receiver of the microwave radiation, which is connected to the multimeter. Working with this mechanism was evaluated as imprecise and the results not satisfactory.

The aim of the invention is to disclose the method for monitoring of spreading of moisture in porous materials and apparatus for its practice, which should allow precise, automatic and repetitional measurement with possibility to pre-set process of the measurement to determine the capillary conductivity coefficient.

The invention is defined in the claims.

Description of the drawings

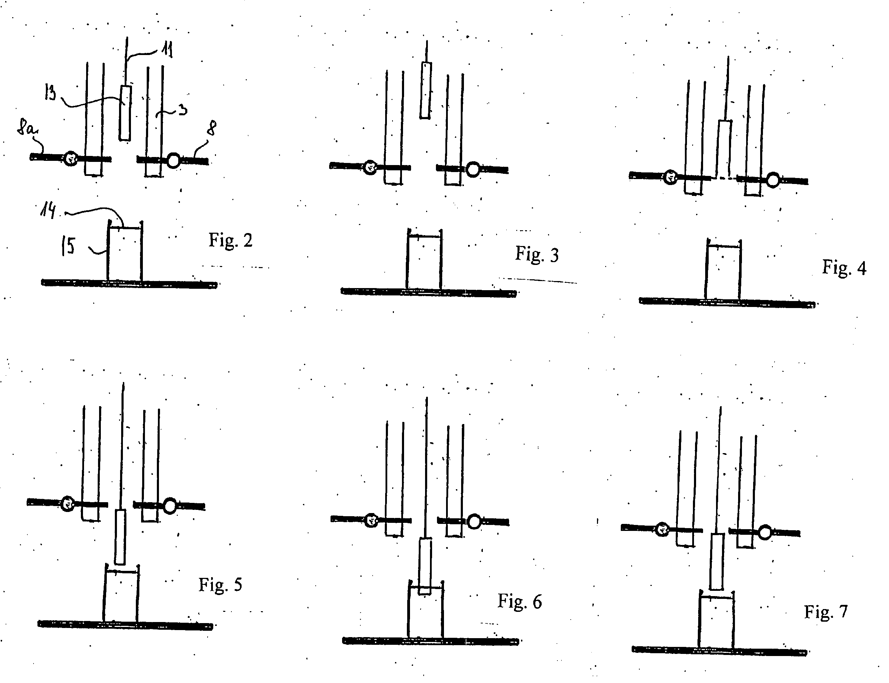

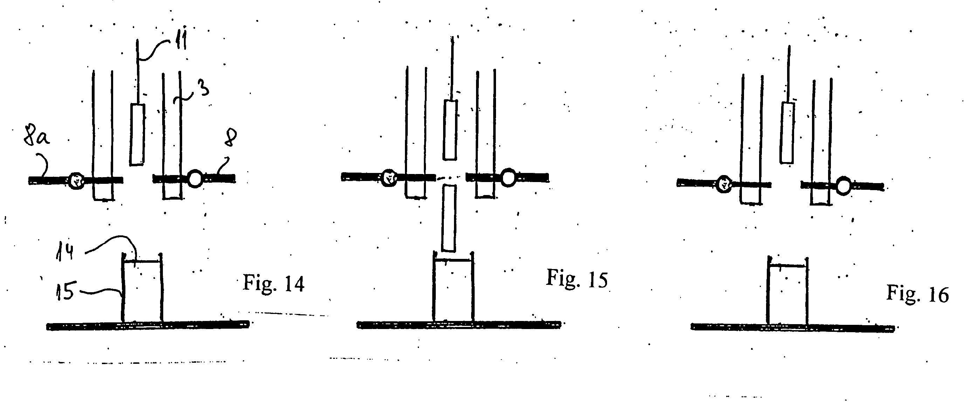

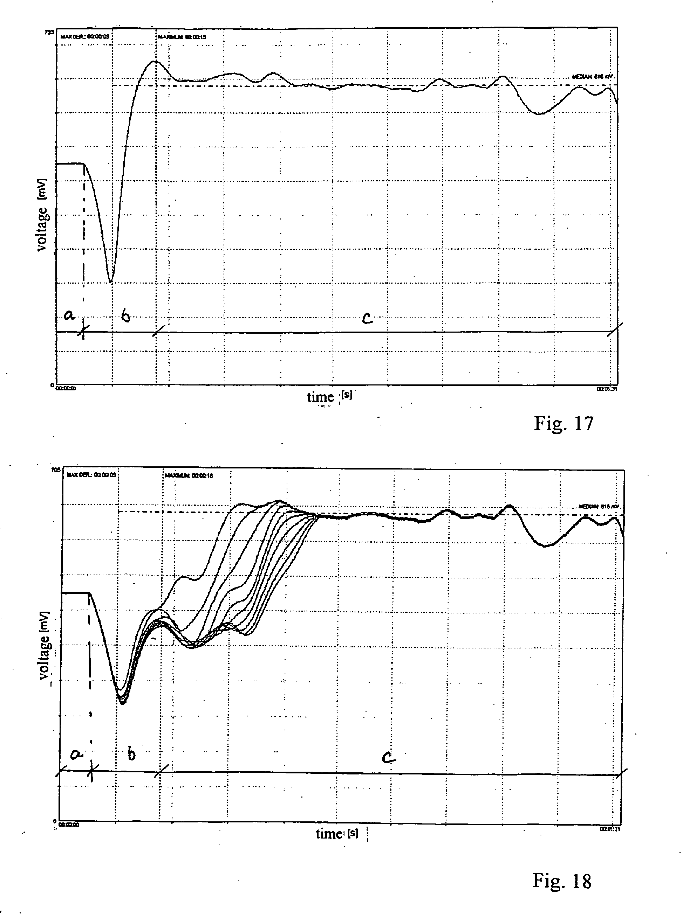

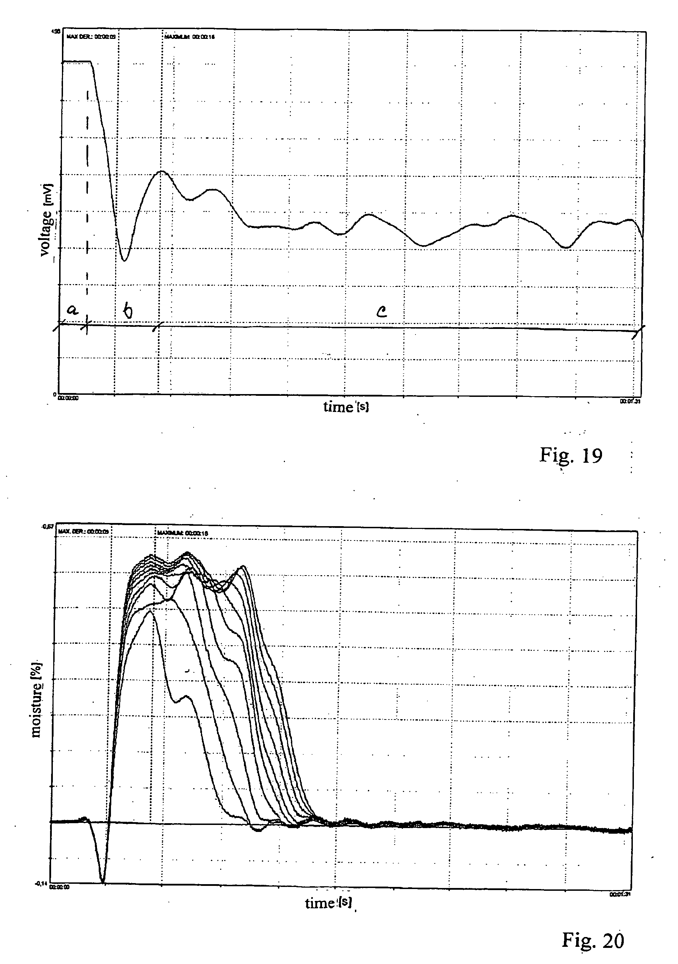

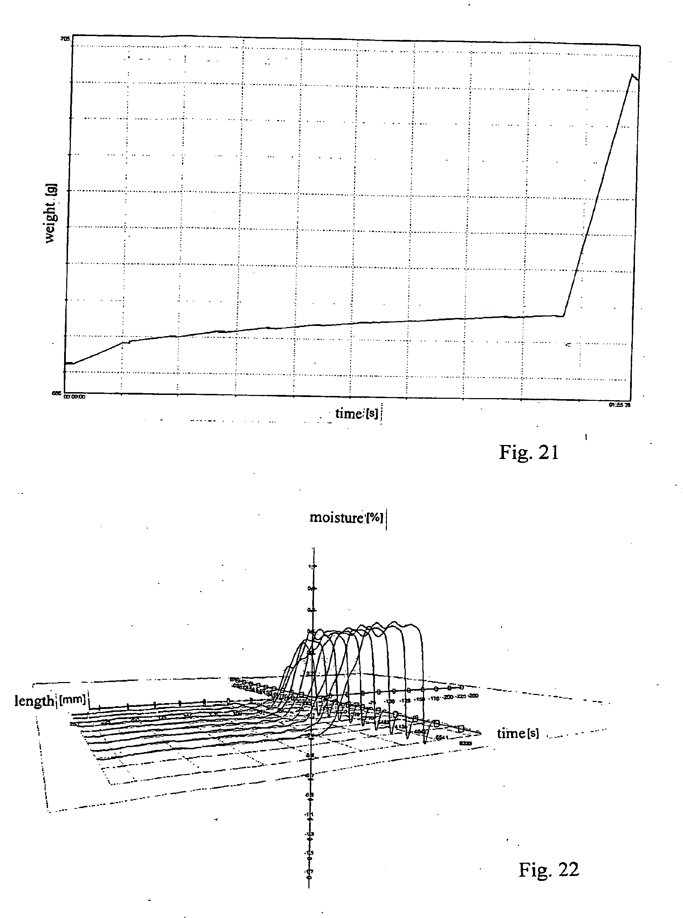

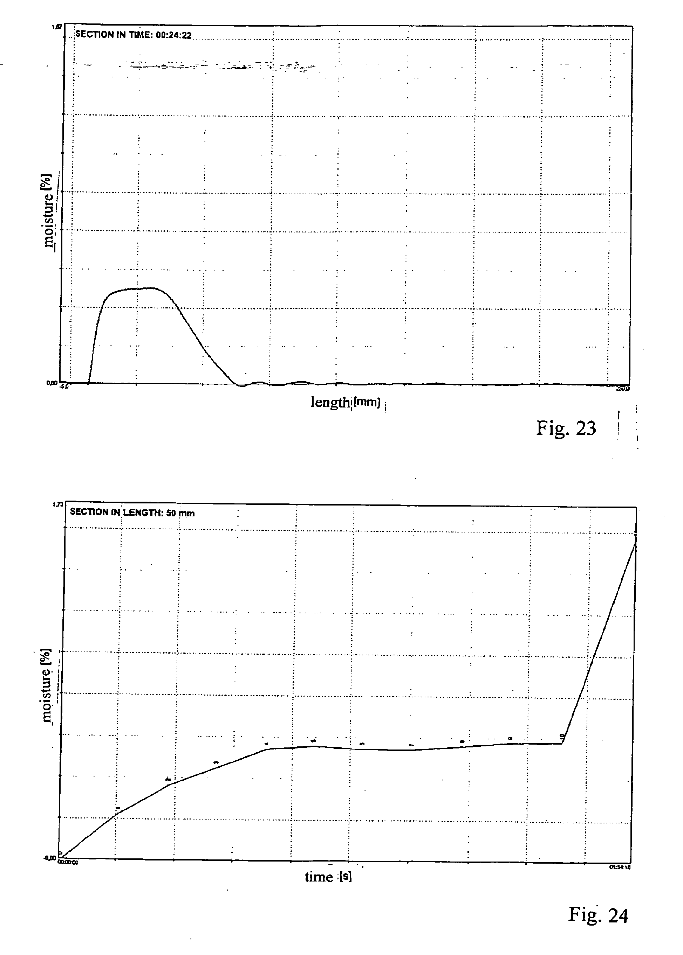

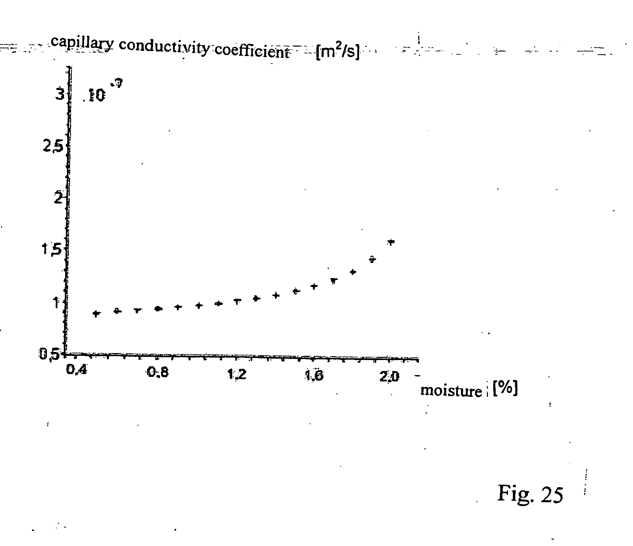

The invention will be further explained by use of drawings, in which Fig. 1a presents a common form of a moisture curve, which creates a moisture forehead of a moisture spreading profile in a material, Fig. 1 presents an apparatus for meassuring of moisture parameters of porous materials according to the invention, Fig. 2 up to Fig. 16 schematically present specific steps of the measuring method according to the invention on the apparatus on Fig. 1, which is illustrated in Fig. 2, Fig. 17 is a graphic illustration of the first calibration measurement, Fig. 18 is a graphic illustration of the applied measuring method, Fig. 19 is a graphic illustration of the second calibration measurement, Fig. 20 presents a graphic summary of processed results of values by particular measurements for determined time intervals as a three-dimensional spreading of physical characteristics, Fig. 21 presents an illustration of a resulting growth of the moisture weight of the specimen as a consequence of its contact with a water level of one of the gained moisture curves, Fig. 22 presents a final result matrix of a three-dimensional illustration of the moisture spreading in an observed specimen based on particular measurements, Fig. 23 presents a moistening curve in a nonstationary state of moistening at a specific time from beginning of moistening, Fig. 24 presents a moistening process by material of a specimen in a chosen cross-section, where the graphic illustration of spreading a humidity weight during moistening of a sample is shown and Fig. 25 is an illustration of the functional relation between the capillary conductivity coefficient on the moisture weight of a specific sample.

Preferred embodiments of the invention

In a construction scheme presented in Fig. 1 it is good to see, that an apparatus 1 intended for observing of moisture diffusion in a porous material according to the invention, consits of a baseplate 2, on which a pair of guiding rails 3 is fixed. A frame 4 of the scale 5 is slidely movable on the guiding rails 3. The rear part of the frame 4 is provided by screw casing, throught which a screw rod 16 is arranged. The screw rod 16 is driven by an engine 6. Top of the screw rod 16 is arranged freely rotatable in an upper casing 24. The screw rod 16 presents a positioning elements intended for height reconfiguration of the frame 4 and also to height reconfiguration of a specimen 13. The specimen 13 is hung on a hanger 11, which is fixed to the digital scale 5, which is arranged above it. The specimen 13 is fixed by a holder 12. A reservoir 14, which is full of water 15, is arranged below the baseplate 2. Waveguides 8 and 8a of a microwave radiation system are placed above the reservoir 14 in the lower part of the baseplate 2. The transmitting waveguide 8 is on its side provided by a source 7 of the radiation. In presented case the source 7 is represented by Gunn-diode, which is connected to a voltage supply, which is necessary for function of the Gunn-diode. In front of the source 7 of the radiation a transmitting antenna 9 is arranged. The receiving waveguide 8a is provided by receiving antenna 10. Both waveguides 8, 8a are stationary fixed on the baseplate 2 as one unit. The waveguides 8, 8a are made of a hollow metal profile. The specimen 13 is arranged between mutually counter-possitioned ends of the waveguides 8, 8a. The receiving waveguide 8a is provided with receiver 10 of microwaves. The receiver 10 is connected to a voltmeter 18, intended to read the value of an intensity change of an output radiation. The voltmeter 18 is connected to a computer 23 to watch the results, for example in the form of curves - it will be descibe hereinafter. Transfer of indicated values is also done by comunication program, intended for data's reading from the display of the digital scale and the software for thr voltmeter 18. The connection between the computer 23 and the motor is performed by comunication motor cable 19. A connection between the computer 23 and the scale 5 is performed by comunication scale cable 22. A connection between the computer 23 and the waveguide 7 is performed by comunication relay cable 21 provided by a relay 17 intended for switch on/off the device. A connection between the computer 23 and the waveguide 7 is performed by comunication voltmeter cable 20 provided by above mentioned voltmeter 18.

It is good to remind some general characteristics of the tested materials before description of the function of the apparatus according to the invention. The specimen 13 absorbs a humidity up to the specific height for a specific lenght of time, during moistening of the forehead of the specimen 13 by its contact with the water level 15. It defines so-called moistening forehead profile. The moisture in the specimen is highest next to the water level, whereas it decreases upwards. This change of moisture is subject matter of the examination.

The aim of the measurement is to find out the moisture gradient. By knowledge of the time's and moisture's space distribution, so by knowledge of the moisture gradient, it is possible to calculate from measured values the values of the capillary conductivity coefficient K.

In Fig.1a parameters, which are necessary for formulation of the capillary conductivity coefficient are presented. The item $t$ is a measuring interval, the moisture gradient is a relationship du/dx. On the axis $x$ is length of the moisture forehead and on the axis $u$ is the moisture weight.

By use of the nonstationar method for determination of the capillary conductivity coefficient it is good to know more moisture curves for different time, for the same specimen from beginning of the experiment (integral method).

In Fig. 2 up to Fig. 15 particular steps of the method according to the invention are described. The process is described thereinafter:

Fig. 2 first step - scaling of the specimen 13

Fig. 3 - position calibration of the specimen 13, which will go up to the most upper position HP, and turning on the microwave radiation - the ray $z$

Fig. 4 - fast downwards movement of the specimen 13, searching the waveguides based on a change of voltage, gentle alighting run to the ray $z$. First calibration measurement is done. The start of its through passing is illustrated. The specimen 13 continues by passing through the ray $z$. The matrix $K_1^*$ is determined.

Fig.5 - the specimen 15 passed through the ray, the microwave device is turned off

Fig. 17 is a graphic illustration of the first calibration measurement described by the steps illustrated in Fig. 4 and Fig. 5 - determination of the matrix $K_1^*$ during slow movement of the specimen between the waveguides, where the sign * is a three-dimensional matrix. It concerns the measurement of the specimen in its dried state through its length. It is a graphic ilustration of voltage changes, which are detected by the receiving antenna after passing of the specimen through it, depending on a humidity content in the length of the specimen from its lower end.

Rolling time of the specimen between the waveguides is marked on the horizontal axis in seconds. Detected values of voltage are marked on the vertical axis in millivolts.

It is evident from the graph, that in whole length of the specimen there are constant voltage values. Dispersion of detected values of the voltage in the length of the specimen is negligable. It is caused by structure of the material of the specimen. This "calibration" represents a base for evaluation of subsequent measurements.

The graf illustrates the initional section $a$ of the curve at the time 0 - 4s, which represents "start of movement of the specimen between the waveguides", when transmited radiation without the specimen is detected, and the section $b$ of a curve at the time 4 - 16s, when the specimen is comming between the waveguides. Above mentioned curve's sections are not decisive for future processing. The decisive section is the section $c$.

Fig. 6 - rough searching of the water level during fast driving down of the specimen, based on a weight change of the specimen as a result of contact of its forehead with water.

Fig. 7 - preparation of a precise detection of the water level by fast lifting of the specimen above the water level to keep it from moistening.

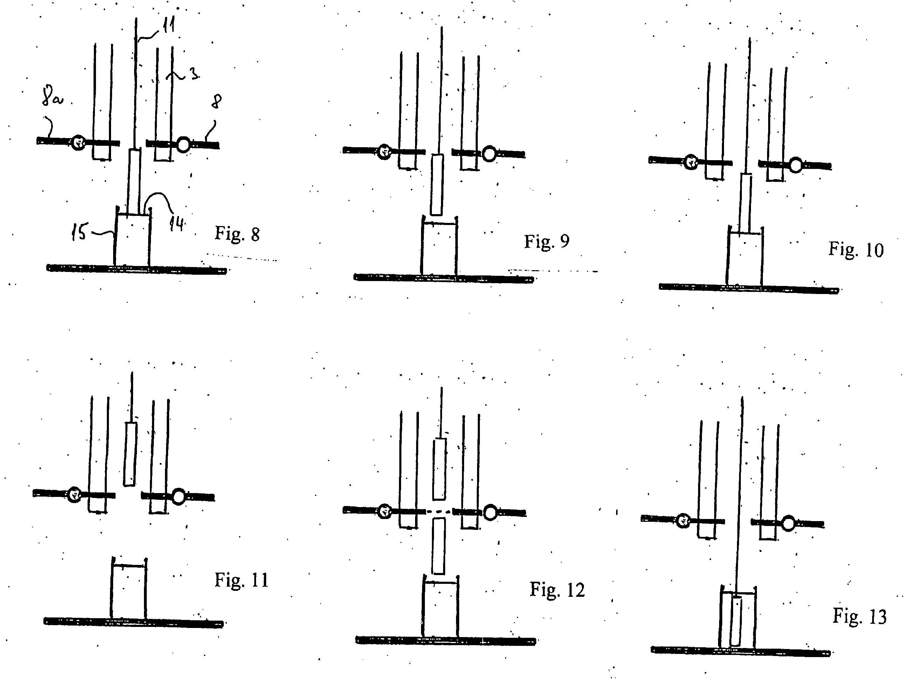

Fig. 8 - precise detection of the water level $h$ based on a weight change of the specimen - slow ride on to the water level.

Fig. 9 - end of the calibration, fast lifting above the water level

Fig. 10 - precise detection of the water level based on rough detection from calibration (illustrated in Fig. 6)

Fig. 11 - preparation of a measurement - fast lifting above the waveguides

Fig. 12 - turning on the microwave radiation $z$, measuring is in progress (the specimen is passing through the Gunn diode), determination of the matrix An*, slow down ride of the specimen during its passing through the waveguides

Repeating of the measurement follows, so again precise search of the water level (Fig. 10), fast lifting over the waveguides, turning on the microwave radiation $z$ (Fig. 11), measurement, determination of the matrix An*, slow down ride (Fig. 12)-each passing through the waveguides represents the obtaining the moisture curve NK.

In Fig. 18 a graphic illustration of the measuring is presented - determination of the matrix An* during slow movement of the specimen 13 between the waveguides 8, 8a - see the situation described in Fig. 10 up to Fig. 12. It concerns measurement of the specimen during its moistening caused by the contact between its the low surface and the water level, for predeterminated time interval, where the sign * is a three-dimensional matrix. It concerns a grafic illustration of measured values of the detected voltage change in its length depending on moisture, which is rising into the specimen in the time of passing of the specimen through the waveguides.

The horizontal axis ilustrates the noticed traversing time of the specimen by individual measurement in seconds, whereas each measurement is starting at the time zero (so it means relative time). However each measurement, in accordance to the absolut time, is proceded after predetermined time intervals of moistening of the specimen (e.g. 15 min interval) - see Fig. 4.

The measured values of voltage of individual measurements are noticed on the vertical axis in milivolts. In the figure ten subseqent measurements are presented, however different number of measurements is possible.

It concerns the measurement of the specimen during its moistening.

The initional sections $\underline{a}$ of the curve (at the time 0 - 4s), when the radiation is provided without the specimen, and the raising sections $\underline{b}$ (at the time 4 - 16s), when the specimen is passing through the waveguides, are kept in the graph. These initional and raising sections are not used for further processing. Decising section is the section $\underline{c}$. The measured values are base for further calculation processing.

Fig. 13 - dipping of the specimen, slow down ride of the specimen below the water level, moistening, keeping the specimen below the water level for the predetermined time

Fig. 14 - preparation of second calibration measurement, fast ride of the specimen above the waveguides

Fig. 15 - turn on the radiation $\underline{z}$, second calibration measurement is proceeded; here the specimen is illustrated above the ray before its passing through, the specimen continues by its passing through the ray $\underline{z}$ to the low position, which is illustrated there in this figure, determination of the matrix $K_2{}^*$ is provided

In Fig. 19 grafic illustration of second calibration measurement is presented - determination of the matrix $K_2{}^*$ - slow movement of the specimen between the waveguides - see the description of that process in Fig. 14 and Fig. 15. The data output of the measurement of the specimen after its moistening caused by its dipping below the water level for the predetermined time, where the sign * is a three-dimensional matrix.

This is graphic illustration of the value of detected voltage depending on the change of electromagnetic microwave radiation intensity in its lenght at the time of passing the specimen between the waveguides. The horizontal axis presents measured movement time of the specimen in seconds. The values of the measured voltage is noted on the vertical axis in the milivolts. It concerns measurement of the specimen at the wet condition after its dipping below the water level for the predetermined time - Fig. 13 - dipping of the specimen $\underline{13}$ below the water level and keeping the specimen below the water level.

The initional sections $\underline{a}$ of the curve (at the time 0 - 4s), when a radiation is provided without the specimen, and the raising sections $\underline{b}$ (at the time 4 - 16s), when the specimen is passing between the waveguids are kept in the graph. These initional and raising sections are not used for further processing. The decising section is the section $\underline{c}$.

Fig. 16 - another scaling of the specimen to find out an increase of its weight. It is the last parameter, which is necessary to find out - it is analogic to the situation ilustrated in Fig. 2. A new cycle with the specimen, which has an increase of a weight caused by absorbing of the liquid during the previous cycle can start.

Evaluation:

Processing of the matrixes ($K_1$, $A_1$, $A_2$.........., $A_n$ , $K_2$,)

Drawing of the graphs - see Fig. 17 up to Fig. 22

Determination of the capillary conductivity coefficient by the moisture gradient K - e Fig. 23

The sign * is a three-dimensioned matrix:

1. time
2. location of the specimen

In Fig. 20 graphic interpretation of a moisture weight values is illustrated. The values were obtained by conversion a relation between the intensity change of electromagnetic microwave radiation and spreading of the moisture content during moistening. It is defined by moving time of the specimen between the waveguides in its length. The figure presents ten subsequent measurements, however different number of measurements is also possible. The values of the moisture weight were determinated on base on voltage changes of individual measurements and by subsequent integration correction.

A measured movement time of the specimen during individual measurements (each measurement is starting in time zero, so it means relative time) is noticed on the horizontal axis in seconds. Each measurement, in accordance to the absolute time, proceeds after predetermined time intervals of the moistening of the specimen (e.g. 15 min. intervals) - see Fig. 4.

On the vertical axis the weight of the moisture of the specimen during individual measurements is noticed in percentage.

The initional sections (at the time 0 - 4s), when the radiography proceeds without the specimen, and the raising sections (at the time 4 - 16s), when the specimen is subsequently passing between the waveguids, are kept in the graph. These initional and raising sections are not used for further processing.

In Fig. 21 a graphic presentation of a final increase of a humidity weight of the specimen, according to the continual moistening at the time of contact between the low surface of its cross section and the water level is presented.

The horizontal axis represents measuring time (in this case it is absolute time). The values of moisture weight of the specimen are noted on the vertical axis. Determination of the values is based on subtraction of weight of the specimen for a relevant measurement (partially moistening of the specimen) from weight of the specimen at a dry state - see Fig. 2: Determination of a dry specimen's weight - scaling.

In Fig. 22 a depiction of a three-dimensional visualisation of the final matrix of moisture spreading in a searched specimen, which is based on individual measurements, is presented. It is an illustration of a graphic results output of processed values of single measurements for a predetermined time interval, which is formed as a three-dimensioned spreading of physical values. First horizontal axis represents time, it means the measurement time in seconds (it is absolute time). Second horizontal axis represents lenght of the measured specimen in millimeters. The value of a moisture weight is noted on the vertical axis in dependence on moisturing time and the position of a moisturing profile of the measured specimen. Above mentioned three-dimensional graph is possible to display as a two-dimensional graph by cross-section in any position. The two-dimensional form is a subbase for counting the capillary conductivity coefficient - see following graphs.

Fig. 23 presents a moisture curve of moistening in a nonstationar moistening state for predetermined time from beginning of moistening. One of all obtained moisture curves is presented as a graphic illustration of spreading of a moisture weight at the specimen length. It is time's cross-section of a previous graph, which is presented in Fig. 22. The time in this case was selected as 24 min and 22 sec. The graph presents a profile of a moisture forehead at the selected time.

Fig. 24 presents a moistening process of the specimen for a selected cross-section. It is a graphic illustration of a spread moisture weight at the moisture time of the specimen. It concerns a longitidinal cross-section of the graph in Fig. 22. The value of the constant possition for that case is selected as 50 mm. That graph expresses the process of the change of moisture weight in the specimen at the specific determined position of the specimen during its moistening at contact time of the low surface with the water level.

Fig. 25 presents a functional relation between the capillary conductivity coefficient and a humidity weight of the specimen. The specimen is from a ceramic burnt material characterised by:

Size of the specimen: length 250 mm, width 60 mm, thickness 20 mm

Moistening medium: water
Temperature of the water: 20°C
Room temperature: 22°C
Beginning weight: 668,9 g
Number of cycles: 10
Time interval between the cycles: 15 min
Lenght of dipping between the cycles: 15 min
Final weight: 702,2 g

Industrial applicability

Thanks to application of the above mentioned method and using of the measured values it is possible to obtain the functional relation between the capillary conductivity coefficient and a humidity weigth for different materials.

Normally, the capillary conductivity coefficient occurs in a thermal technical calculation as an input parameter. However finding out its value for a specific material is extremly problematic. If it is possible to specify it, for example by charts, it is referred only by its only one value, respectively by value in the dry state. Based on the above mentioned method it is possible to put the capillary conductivity coefficient to the thermal technical calculation as the functional relation on the humidity weight which enables to obtain obviously more precise calculations.

**Claims**

1.  Apparatus for measurement of moisture parameters of porous materials comprising a hanger (11) for the experimental specimen (13), wherein the hanger (11) is fixed to a digital scale (5) above it, below the hanger (11) a container (14) with water (15) is arranged and above the container (14) below the hanger (11) waveguides (8, 8a) of microwave radiation are arranged, wherein on the rim of the waveguide (8) a source (7) of the radiation is arranged, which is a Gunn diode, which is connected to a voltage source, wherein in the front of the source (7) of the radiation on the transmitting waveguide (8) a transmitting antenna (9) is arranged and on the receiving waveguide (8a) a receiving antenna (10) is arranged and both waveguides (8, 8a) are stationarily fixed as one unit, wherein from the waveguides to a computer and from the scale to the computer cables are installed, wherein between the waveguide and the computer a voltmeter (18) is arranged and the receiving antenna (10) is connected to the voltmeter (18) **characterised in that** it further comprises a baseplate (2) on which pair of guiding rails (3) is fixed, wherein on the guiding rails (3) the scale's frame (4) is slideably arranged, wherein the rear side of the scale's frame (4) is provided by a screw casing, through which a driving rod (16) is arranged, which is anchored in an engine (6) and the driving rod (16) is on its upper side freely rotatably arranged in an upper casing (24), wherein the computer (23) and the engine (6) are connected by a communication motor cable (19), and the computer (23) and the source (7) of the radiation (6) are connected by a relay communication cable (21), which is provided with a relay (17) intended for switching on/off of the device.

2. Method for monitoring of moisture spreading in porous materials by the apparatus according to claim 1 **characterised in that**, it comprises the following steps:

- scaling of the specimen;
- calibration of a specimen's location, which is lifted to the upper extreme position, and turning on of the microwave radiation;
- fast downward ride of the specimen, searching of the waveguides based on voltage changes, precise reaching of the ray, whereby a first calibration measurement is realised and then matrix $K_1^*$ is determined;
- turning off of the device after passage of the specimen through the ray;
- rough search of the water level by fast downward ride of the specimen based on weight changes of the specimen caused by its contact with water;
- preparing of precise searching of the water level by fast lifting of the specimen above the water level to avoid sucking of the moisture;
- precise search of the water level based on weight changes of the specimen - slow ride till the water level is reached;
- finalization of calibration, fast lifting of the specimen above the water level;
- precise search of the water level based on rough search calibration;
- preparing of measurement based on fast upward ride above the waveguides;
- turning on the microwave radiation and self measurement at the time of passage of the specimen through the waveguides, whereby the matrix An* is determined;
- repeating of the measurement, thus another precise search of the water level, fast lift of the specimen above the waveguides, turning on the microwave radiation, self measurement, determination of the matrix An*, wherein each passage of the specimen means obtaining of one moisture curve;
- diving of the specimen, slow ride of the whole specimen below the water level, moistening, keeping of the specimen below the water level for sufficient time;
- preparing of second calibration measurement, fast ride of the specimen above the waveguides;
- turning on the radiation, whereby a second calibration measurement is in progress, the specimen continues by its passing through the ray to the low position, whereby matrix $K_2^*$ is determinated
- another scaling of the specimen for determination of the weight increase.

## Patentansprüche

1. Vorrichtung für die Ermittlung der Feuchtigkeitsparameter in porösen Stoffen bestehend aus einer Aufhängung (11) für die Probe (13), wo die Aufhängung (11) zu einer Digitalwaage (5) die befestigt ist, wobei unter der Aufhängung (11) ein Behälter (14)) mit Wasser (15) angerichtet ist und über dem Behälter (14) und unter der Aufhängung (11) Wellenleiter (8,8a) der Mikrowellenstrahlung angeordnet sind, wobei am Ende des Sendewellenleiters (8) eine Radiationsquelle (7) in der Form von Gunn-Diode angeordnet ist, welche zu einer Spannungsquelle angeschlossen ist, wobei vor der Radiationsquelle (7) auf dem Sendewellenleiter (8) eine Sendeantenne (9) angebracht ist und auf dem Empfanswellenleiter (8a) eine Empfangsantenne (10) angebracht ist und beide Wellenleiter (8,8a) stationär als eine Einheit auf dem Rahmen angebracht sind, wobei von den Wellenleiter zum Computer und von der Waage (5) zum Computer Kabel installiert sind, wobei zwischen den Wellenleiter und dem Computer ein Voltmeter (18) installiet ist und die Empfangsantenne (10) auch zu dem Voltmeter (18) angeschlossen ist, **dadurch gekennzeichnet, daß** sie weiter aus einer Grundplatte (2), auf der ein Paar von Führungsschienen (3) angebracht ist, besteht, wobei auf den Führungsschienen (3) ein Waagerahmen (4) gleitend angeordnet ist, wobei auf der hinteren Seite des Waagerahmens (4) eine Schraubhülse angeordnet ist, durch welche eine Triebstange (16), die in dem Motor (6) angeordnet ist, durchläuft und die Triebstange (16) auf der oberen Seite frei rotierend in dem oberen Gehäuse (24) angeordnet ist, wobei der Computer (23) und der Motor (6) mit Motorkommukationskabel (19) und der Computer (23) und die Radiationsquelle (7) mit dem Relaykommukationskabel (21), der mit einem Relay versehen ist, die für Einschaltung/Ausschaltung von der Vorrichtung bestimmt ist, angeschlossen sind.

**2.** Verfahren zur Ermittlung der Feuchtigkeitsverbreitung in porösen Stoffen mit der Vorrichtung nach dem Anspruch 1, **dadurch gekennzeichnet, daß** sie folgende Schritte beinhaltet:

- Wiegen der Probe;
- Kalibration der Probelage, wobei die Probe zur höchsten extremen Position aufgefahren ist und Einschaltung der Microwellenradiation;
- Schnelle Bewegung der Probe nach unten, Suche der Wellenleiter aufgrund der Voltageänderungen, genaue Anfassung von dem Strahl, wobei die erste Kalibration folgt und die Matrize $K_1^*$ bestimmt ist;
- Ausschaltung der Vorrichtung nach der Durchfahrt der Probe durch den Strahl;
- Grobsuche des Wasserspiegels durch schnelle Fahrt der Probe nach unten aufgrund der Gewichtsänderung der Probe, die durch den Kontakt mit Wasser verursacht ist;
- Vorbereitung der Genausuche des Wasserspiegels durch schnelle Fahrt der Probe nach oben über das Wasserspiegel zur Vermeindung der Feutigkeitsansaugung;
- Genausuche des Wasserspiegels aufgrund der Gewichtsänderung der Probe-langsame Fahrt bis das Wasserspiegel erreicht ist;
- Beendigung der Kalibration, schnelle Fahrt der Probe nach oben über das Wasserspiegel;
- Genausuche des Wasserspiegels aufgrund der Grobsuchekalibration;
- Vorbereitung des Messens aufgrund der schnellen Fahrt der Probe nach oben über die Wellenleiter;
- Einschalten der Microwellenradiation und eingentliches Messen zur Zeit der Durchgang der Probe durch die Wellenleiter, wobei die Matrize An* bestimmt ist;
- Wiederholung des Messens, aslo eine andere Genausuche des Wasserspiegels, schnelle Fahrt der Probe nach oben über die Wellenleiter, Einschalten der Microwellenradiation, eingentliches Messen, Bestimmung der Matrize An*, wobei jede Durchfahrt der Probe ein Erwerben von einer Feuitigkeitskurve darstellt;
- Eintauchen der Probe, langsame Fahrt der ganzen Probe unter das Wasserspiegel, Auffeuchtung, Belassen der Probe unter dem Wasserspiegel für eine ausreichende Zeit;
- Vorbereitung des zweiten Kalibrationsmessens, schnelle Fahrt der Probe nach oben über die Wellenleiter;
- Einschalten der Microwellenradiation, wobei das zweite Kalibrationsmessen im Gange ist, die Probe geht weiter mit dem Durchgehen durch den Strahl zu der unteren Position, wobei die Matrize $K_1^*$ bestimmt ist;
- Ein weiteres Wiegen der Probe zur Bestimmung des Gewichtszuwachs;

## Revendications

**1.** Appareillage de mesure des paramètres d'humidité de la masse poreuse comportant la suspension (11) de l'échantillon soumis à l'essai ladite suspension étant fixée sur une balance numérique (5) en-dessus d'elle, sous la suspension se trouve un réservoir (14) contenant de l'eau (15), en-dessus du réservoir (14) sous la suspension étant disposés les guides d'ondes stationnaires (8,8a) du rayonnement micro onde étant donné qu'un guide d'onde (8) comporte sur son extrémité la source (7) de rayonnement conçue sous forme de la diode Gunn connectée à la source de tension devant la source de rayonnement (7), sur le guide d'onde émetteur (8) étant disposée une antenne d'émission (9) et sur l'autre guide d'onde de réception (8a) se trouve l'antenne de réception, (10) et à partir des guides d'ondes et de la balance sont installés les câbles de l'ordinateur entre le guide d'onde et l'ordinateur se trouvant le voltmètre (18), et l'antenne de réception (10) est reliée au voltmètre (18), **caractérisé en ce que** le réservoir (14) est conçu comme élément immobile, l'appareillage étant constitué par ailleurs de la plaque principale (2) sur laquelle est fixée de manière verticale une paire des barres de guidage verticales (3) sur les barres de guidage (3) étant disposé de manière glissante le châssis (4) de la balance (5) le châssis (4) de la balance (5) étant pourvu par derrière d'une douille à vis traversée par une tige de commande filetée (16) posée dans le moteur (6) la tige de commande (16) étant dans sa partie supérieure tournante disposée dans la douille supérieure (24) étant donnée que l'ordinateur (23) est relié avec le moteur (6) par un câble de communication motorisé (19) et l'ordinateur (23) est relié à la source (7) de rayonnement par un câble de communication à relais (21) pourvu d'un relais (17) pour la mise en marche/mise hors de la marche du rayonnement.

**2.** Procédé de suivi de la propagation de l'humidité dans les masses poreuses sur l'appareillage selon la revendication 1, **caractérisé en ce qu'**il comporte les opérations suivantes :

- pesage de l'échantillon;
- calibrage de la position de l'échantillon qui sortira dans sa position d'extrémité supérieure et mise en marche du rayonnement micro onde;
- mouvement rapide de l'échantillon vers le bas, recherche des guides d'ondes sur la base du changement de la tension, butée fine sur le rayon lors de laquelle est effectué le premier mesurage de calibrage et la matrice $K_1^*$ est définie;

- mise hors de la marche après le passage de l'échantillon par le rayon;
- recherche grossière du niveau par l'intermédiaire d'un mouvement rapide vers le bas, sur la base des variations du poids de l'échantillon en conséquence du contact de son front avec l'eau;
- préparation d'une recherche fine du niveau par l'intermédiaire d'un relèvement rapide de l'échantillon au-dessus du niveau pour qu'il n'ait pas le temps d'absorber l'humidité;
- recherche fine du niveau sur la base des variations du poids de l'échantillon-mouvement lent jusqu'au niveau;
- achèvement du calibrage, sortie rapide au-dessus du niveau;
- recherche fine du niveau sur la base de la recherche grossière du calibrage;
- préparation du mesurage consistant en sortie rapide au-dessus des guides d'ondes;
- mise en marche du rayonnement micro onde et mesurage proprement dit lors du passage de l'échantillon par les guides d'ondes en déterminant la matrice An*;
- mesurage répété, donc une nouvelle recherche fine du niveau, sortie rapide au-dessus des guides d'ondes, mise en marche du rayonnement micro onde, mesurage proprement dit, détermination de la matrice $A_n^*$, chaque passage représentant l'obtention de la courbe d'humidité;
- immersion de l'échantillon, mouvement lent de l'échantillon en-dessous du niveau, humidification, attente avec l'échantillon en-dessous du niveau pendant le temps défini;
- préparation du deuxième tour du mesurage de calibrage, mouvement rapide en-dessus des guides d'onde;
- mise en marche du rayonnement, réalisation de second mesurage de calibrage, l'échantillon poursuit sa traversée à travers le rayon en position inférieure, détermination de la matrice $K_2^*$;
- nouveau pesage de l'échantillon dans le but de déterminer l'accroissement du poids.

Fig. 1

Fig. 1a

Fig. 2

Fig. 3

Fig. 4

Fig. 5

Fig. 6

Fig. 7

EP 2 498 080 B1

Fig. 8

Fig. 9

Fig. 10

Fig. 11

Fig. 12

Fig. 13

Fig. 16

Fig. 15

Fig. 14

Fig. 17

Fig. 18

Fig. 19

Fig. 20

Fig. 21

Fig. 22

SECTION IN TIME: 00:24:22

moisture [%]

length [mm]

Fig. 23

SECTION IN LENGTH: 50 mm

moisture [%]

time [s]

Fig. 24

Fig. 25

**EP 2 498 080 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CZ 301475 B1